# EUROPEAN PATENT APPLICATION

(11) **EP 3 792 616 A1**
(43) Date of publication of application: **17.03.2021**
(21) Application number: 19799691.1
(22) Date of filing: 06.05.2019
(51) Int. Cl.: G01N 21/01, G01N 21/17

(54) **SOLID-PHASE SURFACE AND SOLUTION MOTION MODE AND MOTION DEVICE**

(30) Priority: 09.05.2018 CN 201820685326 U; 09.05.2018 CN 201810438306
(71) Applicant: Shanghai Jiaotong University, Shanghai 200240 (CN)
(72) Inventor: YANG, Tian, Minhang District, Shanghai 200240 (CN); HE, Xiaolong, Minhang District, Shanghai 200240 (CN); WANG, Yalin, Minhang District, Shanghai 200240 (CN)
(74) Representative: Canzler & Bergmeier Patentanwälte Partnerschaft mbB
(86) International application number: PCT/CN2019/085622
(87) International publication number: WO 2019/214563

(57) **Abstract**

A solid-phase surface and solution motion mode and motion device. In an interaction process of a solid-phase surface and a target object, the target object is dissolved or dispersed into a solution, the solid-phase surface and the solution make a relative motion, and the relative motion comprises a relative motion perpendicular to the solid-phase surface so as to improve at least one of the binding rate, dissociation rate, binding uniformity, binding directivity, and binding compactness of the target object and the solid-phase surface. Compared With the existing motion mode parallel to a sensing surface, the motion mode can effectively improve the binding efficiency and dissociation efficiency of a ligand and an analyte at the same relative motion velocity of a sensor and the solution.

## Description

### Technical Field

The present disclosure belongs to the field of biosensing detection, and in particular, to a solid-phase surface and solution motion mode and motion device.

### Background

The measurement of kinetic and thermodynamic processes of biomolecular interactions is an important cornerstone in life science research and drug development. Taking antibody drugs as an example, the research and development of each new drug involves thousands of samples, and goes through processes such as target and antibody screening, engineering cell line construction, clinical blood drug pharmacokinetics, immunogenicity detection, and industrial quality control. Each of the processes requires rigorous biomolecular interaction testing to make an accurate evaluation. Label-free sensing is one of the key technologies, which is at a rapid development stage in application and industrial scale.

Compared with traditional detection methods represented by enzyme-linked immunosorbent assay, the label-free sensing maintains the natural characteristics of the samples and greatly saves the time and labor of the operators. At the same time, label-free sensing has great value in scientific research and drug screening due to the real-time measurement of the molecular interaction process. Currently, the most commonly used label-free sensing technology is based on surface plasmon resonance (SPR). In the second half of 2016, SPR was included in the United States Phannacopoeia and Japanese Pharmacopoeia as an immunological test method for preclinical evaluation of drugs. SPR are surface waves formed by the coupling between the free charge oscillation on the metallic surface and the light field. The resonant coupling condition of SPR with the incident light is very sensitive to the density of the material attached to the metallic surface. When a specific ligand is immobilized on the metallic surface to capture analyte molecules, the changes in the SPR optical signal may be used to determine the binding conditions of the analyte to the ligand, to obtain information such as the concentration, specificity, affinity and kinetic parameters of the analyte.

Almost all traditional SPR products, represented by Biacore's instrument series and supporting sensing chips, are based on microfluidic injection. In such a sample injection method, the ligand is immobilized on the sensing chip, the sample solution contains the analyte to be tested, and the sample solution flows in the microfluidic channel at a speed class of up to m/min in a direction parallel to the sample surface. The microfluidic injection method can constantly update the sample solutions and wash the surface of the sensing chip, improving the capture efficiency of analytes by the ligand, and solving the problem that the analyte and ligand are difficult to bind or dissociate due to slow diffusion and other reasons. For experiments with low concentration sample solution and slow analyte mass transfer speed, it is necessary to increase the flow velocity to obtain a large ligand-analyte binding capacity and good kinetics measurement results of ligand-analyte interaction.

Unlike the SPR sensing chip with microfluidic injection method, in Octet products of ForteBio, an end facet of a thick optical cable serves as a surface of the sensor, the ligand is immobilized on the surface, and the sample solution containing the analyte is placed in an open container such as a multi-well plate. Compared with SPR chip products improving ligand-analyte binding efficiency by increasing microfluidic flow speed, the sample solution container of Octet circularly rotates and oscillates in a plane parallel to the end facet (solid-phase surface) of the thick optical cable, with the rotation speed up to 1000rpm. However, since the rotation speeds of the sample solution are different at different positions, the closer the sensing surface is to the rotation center, the less the efficiency of the ligand-analyte interaction is improved. Therefore, if the positioning of sensors, samples, rotary table and other components cannot be accurately guaranteed, such motion mode will increase the error of experimental results. Besides, the effect of rotary oscillatory motion mode on the molecular arrangement and spatial orientation on the sensing surface is also different from that of unidirectional flow, which may result in different experimental results.

These two motion modes of the sensor and sample solution, namely 1) the sample solution flows unidirectionally in a direction parallel to the solid-phase surface, and 2) the sample solution rotates around the center in a plane parallel to the solid-phase surface, have been widely applied in the experiments of label-free biomolecule interaction for more than 10 or 20 years, respectively. Anew motion mode of the sensor and sample, which can further increase the ligand-analyte binding efficiency, can improve the sensitivity of the biomolecule interaction experiments and the effect of kinetic measurements. This is especially important for the detection of low concentration and small molecular weight.

Based on the above, it is necessary to provide a motion mode of the sensor solid-phase surface and the analyte solution that can effectively improve the ligand-analyte binding efficiency and dissociation efficiency.

### Summary

The present disclosure provides a solid-phase surface and solution motion mode, to solve the problems of unsatisfactory binding rate and dissociation rate between the solid-phase surface and the target object in the solution.

The present disclosure provides a solid-phase surface and solution motion mode. During an interaction of a solid-phase surface and a target object, the target object is dissolved or dispersed into a solution, the solid-phase surface and the solution move with respect to each other, and the relative motion includes a relative motion perpendicular to the solid-phase surface, so as to improve at least one of the binding rate, dissociation rate, binding uniformity, binding directivity, and binding compactness of the target object to the solid-phase surface.

Preferably, in the binding and dissociating processes of the solid-phase surface to the target object, the relative motion of the solid-phase surface and the solution includes a unidirectional forward motion perpendicular to the solid-phase surface, so as to improve at least one of the binding rate, dissociation rate, binding uniformity, binding directivity, and binding compactness of the solid-phase surface to the target object.

Preferably, in the binding and dissociating processes of the solid-phase surface and the target object, the relative motion of the solid-phase surface and the solution includes reciprocating forward motion and backward motion perpendicular to the solid-phase surface, so as to improve at least one of the binding rate, dissociation rate, binding uniformity, binding directivity, and binding compactness of the solid-phase surface to the target object.

Preferably, the relative motion includes a combination of the relative motion perpendicular to the solid-phase surface and the relative motion parallel to the solid-phase surface.

Further, the relative motion perpendicular to the solid-phase surface and the relative motion parallel to the solid-phase surface are simultaneously performed.

Further, the relative motion parallel to the solid-phase surface includes one of a unidirectional parallel motion, a bidirectional reciprocating parallel motion, and a rotational parallel motion.

Preferably, the relative motion of the solid-phase surface and the solution includes one of only the motion of the solid-phase surface, only the motion of the solution, and simultaneous motions of the solid-phase surface and the solution.

Preferably, the relative perpendicular motion velocity of the solid-phase surface and the solution is no less than 1/10 of the Brownian motion velocity of the target object in the solution, so as to increase the collision probability of the solid-phase surface and the target object.

Preferably, the target object includes a large-particle analyte, and the large-particle analyte is selected from a group consisting of metallic particles, exosome particles, cells, quantum dots, and medium particles, or a combination thereof.

Preferably, the solid-phase surface includes a ligand in the biomolecular interaction and a sensor surface for detecting the biomolecular interaction. The ligand is immobilized on the sensor surface. The target object includes an analyte in the biomolecular interaction, and the analyte is dissolved or dispersed into the solution. The binding and dissociation of the target object to the solid-phase surface is the binding and dissociation of the analyte to the ligand.

Further, the sensor includes one of a sensor on an optical fiber end facet and a sensor on an optical cable end facet. The end facet is approximately perpendicular to the optical wave transmission direction of the optical fiber or optical cable adjacent to the sensor. The sensor on an optical fiber end facet includes a sensor on the end facet of the optical fiber based on surface plasmon resonance, and the sensor on an optical cable end facet includes a sensor on the end facet of the optical cable based on biological membrane layer interference.

The present disclosure further provides a solid-phase surface and solution motion mode. In an interaction process of a solid-phase surface and a target object, the target object is dissolved or dispersed into a solution, the solid-phase surface and the solution move with respect to each other, and the relative motion includes a non-central rotational reciprocating relative motion. The direction of the reciprocating relative motion is parallel to the solid-phase surface, so as to improve the binding rate and dissociation rate of the target object to the solid-phase surface.

Preferably, the solid-phase surface includes a ligand in the biomolecular interaction and a sensor surface for detecting the biomolecular interaction. The ligand is immobilized on the sensor surface. The target object includes an analyte in the biomolecular interaction, and the analyte is dissolved or dispersed into the solution. The binding and dissociation of the target object to the solid-phase surface is the binding and dissociation of the analyte to the ligand. The sensor is selected from a group consisting of a sensor on an optical fiber end facet and a sensor on an optical cable end facet. The end facet is approximately perpendicular to the optical wave transmission direction of the optical fiber or optical cable adjacent to the sensor. The sensor on an optical fiber end facet includes a sensor on the end facet of the optical fiber based on surface plasmon resonance, and the sensor on an optical cable end facet sensor includes a sensor on the end facet of the optical cable based on biological membrane layer interference.

The present disclosure further provides a solid-phase surface and solution motion device, including: a container, the container contains a solution, and a target object is dissolved or dispersed into the solution; a solid-phase surface, the solid-phase surface includes a ligand in a biomolecular interaction and a sensor for detecting the biomolecular interaction, the ligand is immobilized on a surface of the sensor, and the sensor is inserted into the solution; and a drive motor, connected with the container, for implementing a reciprocating motion of the container, and the reciprocating motion includes a motion direction perpendicular to the surface of the sensor, to improve at least one of the binding rate, dissociation rate, binding uniformity, binding directivity, and binding compactness of the target object to the surface of the sensor.

Preferably, the reciprocating motion of the container driven by the drive motor further includes a motion direction parallel to the surface of the sensor. The drive motor simultaneously drives the container to move in a direction perpendicular to the surface of the sensor and in a direction parallel to the surface of the sensor.

Preferably, the drive motor includes a voice coil motor.

Preferably, the container includes any one of a 96-well microtitre plate and a centrifuge tube.

Preferably, the sensor includes one of a sensor on an optical fiber end facet and a sensor on an optical cable end facet. The end facet is approximately perpendicular to the optical wave transmission direction of the optical fiber or optical cable adjacent to the sensor. The sensor on an optical fiber end facet includes a sensor on the end facet of the optical fiber based on surface plasmon resonance, and the sensor on an optical cable end facet sensor includes a sensor on the end facet of the optical cable based on biological membrane layer interference.

As described above, the solid-phase surface and solution motion mode and motion device of the present disclosure has the following beneficial effects:
1) The present disclosure provides a motion mode in which the relative motion of the sensing surface and the solution is perpendicular to the solid-phase surface. Compared with the traditional motion mode in which the relative motion is parallel to a sensing surface, the motion mode of the present disclosure can effectively improve the binding efficiency and dissociation efficiency of a ligand and an analyte at the same relative motion velocity of sensor and sample solution.
2) The perpendicular motion mode of the present disclosure will not cause significant unevenness in the flow rate of the sample solution, and may effectively improve the binding uniformity, binding directivity and binding compactness of the ligand to the analyte.
3) The present disclosure can effectively improve the detection sensitivity of biomolecular interactions, and therefore shows promises for a broad range of applications in biosensing detection.
4) When the perpendicular relative motion velocity of the sensor-sample solution is no less than 1/10 of the Brownian motion velocity of the analyte, such motion mode would significantly increase the probability of ligand-analyte collision. Therefore, for the measurement of the particle analyte with lower Brownian motion velocity and large mass, such as metallic particles, exosome particles, cells, quantum dots and medium particles, the present disclosure can remarkably increase the relative motion velocity of the analyte and the ligand.
5) The sensor-sample solution used in the present disclosure carries out a reciprocating relative motion in a manner parallel to the sensing surface but not rotating around the center, which may significantly improve the ligand-analyte binding efficiency compared to the case without motion. The relative motion velocity in this motion mode does not change with the deviation of the center position of the sensor and sample solution, so that the consistency between different test results is better than that of the central rotational relative motion mode

### Brief Description of the Drawings

FIG. 1 is a schematic diagram of a detection device of the present disclosure.
FIGs. 2 to 4 are schematic diagrams showing several solid-phase surface and solution motion modes according to the present disclosure.
FIG. 5 is a schematic diagram showing horizontal reciprocating motions of an optical fiber.
FIG. 6 is a schematic diagram showing a circular rotary oscillatory motion of a multi-well microtitre plate.
FIG. 7 shows the PPB-SA molecular interaction experiment results according to the present disclosure.
FIGs. 8 to 10 show the experiment results of BPA-hIgG molecular interaction according to the present disclosure.
FIG. 11 is a schematic diagram showing the solid-phase surface and solution motion device according to the present disclosure.

### Description of Reference Numerals

- 10: Light source
- 11: Directional coupler
- 12: Optical fiber
- 13: Sample solution
- 14: Sensor on optical fiber end facet
- 15: Spectrometer
- 16: Analyte
- 21: Container
- 22: Drive motor

### Detailed Description of the Preferred Embodiments

The embodiments of the present disclosure will be described below. Those skilled in the art can easily understand other advantages and effects of the present disclosure according to contents disclosed by the specification. The present disclosure may also be implemented or applied through other different specific implementation modes. Various modifications or changes may be made to all details in the specification based on different points of view and applications without departing from the spirit of the present disclosure.

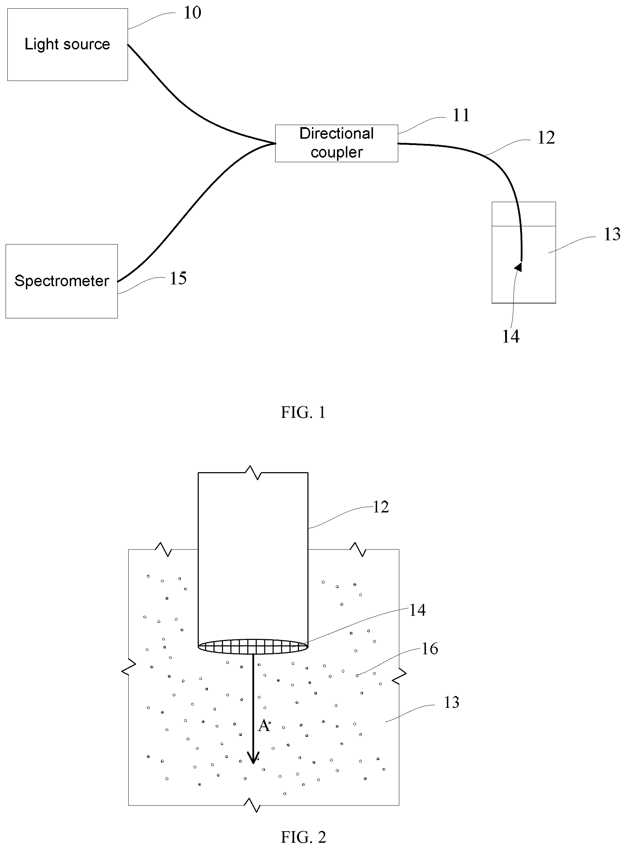

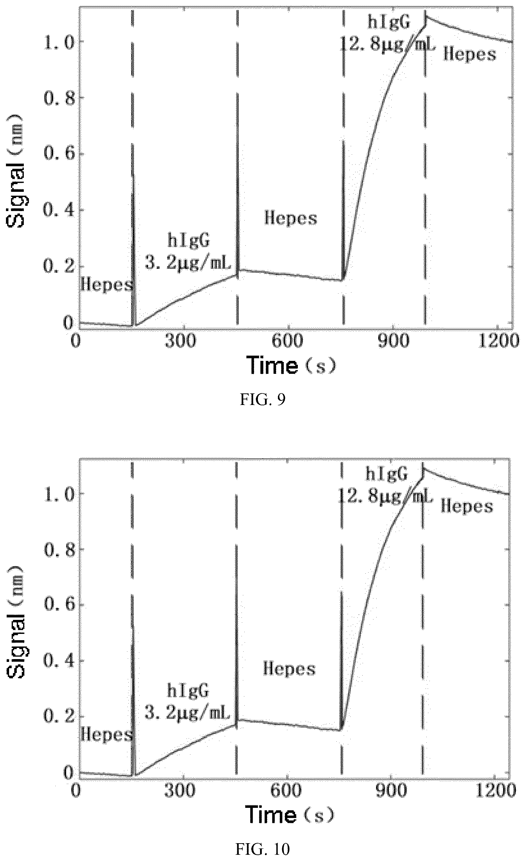

Referring to FIGs 1-11. It needs to be stated that the drawings provided in the following embodiments are just used for schematically describing the basic concept of the present disclosure, thus only illustrating components only related to the present disclosure and are not drawn according to the numbers, shapes and sizes of components during actual implementation, the configuration, number and scale of each component during actual implementation thereof may be freely changed, and the component layout configuration thereof may be more complicated.

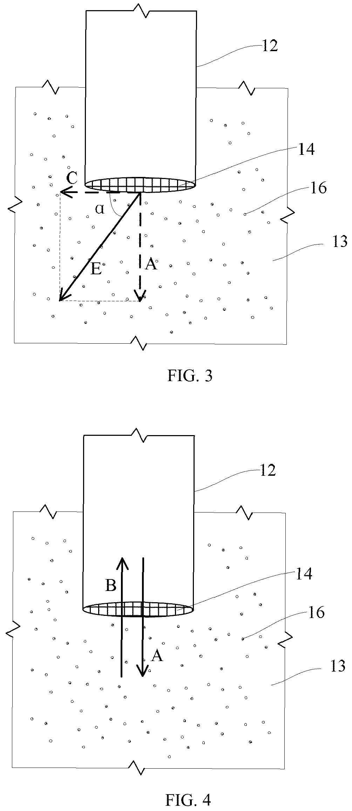

As shown in FIGs. 2 and 4, this embodiment provides a solid-phase surface and solution motion mode. During interaction of a solid-phase surface and a target object, the target object is dissolved or dispersed into a solution, the solid-phase surface and the solution move with respect to each other, and the relative motion includes a relative motion perpendicular to the solid-phase surface, so as to improve at least one of the binding rate, dissociation rate, binding uniformity, binding directivity, and binding compactness of the target object to the solid-phase surface. For example, in the binding and dissociating processes of the solid-phase surface and the target object, the relative motion of the solid-phase surface and the solution may include a unidirectional forward motion A perpendicular to the solid-phase surface (as shown in FIG. 2) or include a reciprocating forward motion A and a backward motion B perpendicular to the solid-phase surface, so as to improve binding rate, binding uniformity, binding directivity, and binding compactness of the solid-phase surface to the target object. In the interaction process of the solid-phase surface and the target object, the reasons affecting the binding effect of the solid-phase surface and the target object include: 1) the solid-phase surface is covered by a quiescent water film, which will slow down the binding and dissociation of the analyte; 2) the diffusion rate of the analyte in the solution will limit the binding rate of the analyte to the solid-phase surface; 3) insufficient exposure of the sites on the ligand that binds to the analyte will reduce the binding rate of the analyte. For the above reasons, it is usually necessary to use a relative motion of the solid-phase surface to the target object, to reduce the thickness of the water film on the solid-phase surface and increase the diffusion rate of the analyte, and help expose the sites on the ligand. Experiments have shown that compared with the parallel motion and the rotational motion, the perpendicular motion of the solid-phase surface and the target object according to the present disclosure has better molecular binding efficiency. The reason may be that the perpendicular motion mode is more effective in scouring the solid-phase surface.

In addition, as shown in FIG. 3, in the binding and dissociating of the solid-phase surface to the target object, the relative motion of the solid-phase surface and the solution may include a combination of the relative motion perpendicular to the solid-phase surface and a relative motion C parallel to the solid-phase surface. If the relative motion perpendicular to the solid-phase surface (such as the forward motion A) and the relative motion C parallel to the solid-phase surface are simultaneously performed, the direction of the relative motion of the solid-phase surface and the solution to be tested may be at an angle α to the solid-phase surface, and the actual relative motion direction is shown as the direction E in FIG. 3. Furthermore, the relative motion parallel to the solid-phase surface includes one of a unidirectional parallel motion, a bidirectional reciprocating parallel motion, and a rotational parallel motion. According to actual needs, an appropriate combination of motion modes can further improve the scouring effect on the solid-phase surface compared to a single perpendicular motion mode, thereby further improving the binding effect of the target object to the solid-phase surface.

It should be noted that the relative motion of the solid-phase surface and the solution may include one of only the motion of the solid-phase surface, only the motion of the solution, and simultaneous motion of the solid-phase surface and the solution. The above-mentioned motion of the solid-phase surface means that the solution does not move but the solid-phase surface moves. The above-mentioned motion of the solution means that the solid-phase surface does not move but the solution moves.

In this embodiment, the solid-phase surface includes a ligand in the biomolecular interaction and a sensor surface for detecting the biomolecular interaction. The ligand is immobilized on the sensor surface. The target object includes an analyte in the biomolecular interaction, and the analyte is dissolved or dispersed into the solution. The binding and dissociation of the target object to the solid-phase surface is the binding and dissociation of the analyte to the ligand. The sensor includes one of a sensor on an optical fiber end facet and a sensor on an optical cable end facet. The end facet is approximately perpendicular to the optical wave transmission direction of the optical fiber or optical cable adjacent to the sensor. The sensor on an optical fiber end facet includes a sensor on the end facet of the optical fiber based on surface plasmon resonance, and the sensor on an optical cable end facet includes a sensor on the end facet of the optical cable based on biological membrane layer interference. It should be noted that although this embodiment focuses on label-free biomolecular interaction experiments, the present disclosure has the same effect on labeled biomolecular interaction experiments. Therefore, the present disclosure is not limited to the examples listed herein. It should be noted that the "approximately perpendicular" includes substantially perpendicular or deviating from perpendicular by a certain angular range. For example, the angular range of the deviation may be between -2° and +2°, but is not limited to the angular range listed here.

The target object includes a large-particle analyte, and the large-particle analyte may include one or more of metallic particles, exosome particles, cells, quantum dots, and medium particles. Further, the relative perpendicular motion velocity of the solid-phase surface (such as the ligand on the sensing surface) and the solution is no less than 1/10 of the Brownian motion velocity of the target object in the solution, so as to increase the collision probability between the solid-phase surface and the target object (such as an analyte). Furthermore, when the relative perpendicular motion velocity of the sensor-sample solution reaches or exceeds the Brownian motion velocity of the analyte, such motion mode would more significantly increase the probability of ligand-analyte collision. Therefore, for the measurement of the particle analyte with lower Brownian motion velocity and large mass, such as metallic particles, exosome particles, cells, quantum dots and medium particles, the present disclosure can remarkably increase the relative motion velocity of the analyte and the ligand. Of course, during the practical application, different relative perpendicular motion velocities may be selected according to different target objects (for example, target objects with different particle sizes), and the present disclosure is not limited to the examples listed herein.

This embodiment further provides a second solid-phase surface and solution motion mode. In an interaction process of a solid-phase surface and a target object, the target object is dissolved or dispersed into a solution, the solid-phase surface and the solution move with respect to each other, and the relative motion includes a non-central rotational reciprocating relative motion. The direction of the reciprocating relative motion is parallel to the solid-phase surface, so as to improve the binding rate and dissociation rate of the target object to the solid-phase surface. Two points on the solid-phase surface form a segment a, two points in the solution form a segment b, with central rotational motion, the angle between the line segment a (and the line segment b changes, instead of the relative position of the line segment a and the line segment b changes while keeping the angle unchanged.

The solid-phase surface includes a ligand in the biomolecular interaction and a sensor surface for detecting the biomolecular interaction. The ligand is immobilized on the sensor surface. The target object includes an analyte in the biomolecular interaction, and the analyte is dissolved or dispersed into the solution. The binding and dissociation of the target object to the solid-phase surface is the binding and dissociation of the analyte to the ligand. The sensor may be a sensor on an optical fiber end facet or a sensor on an optical cable end facet. The end facet is approximately perpendicular to the optical wave transmission direction of the optical fiber or optical cable adjacent to the sensor. The sensor on an optical fiber end facet includes a sensor on the end facet of the optical fiber based on surface plasmon resonance, and the sensor on an optical cable end facet sensor includes a sensor on the end facet of the optical cable based on of biological membrane layer interference. In this embodiment, the sensor-sample solution carries out a reciprocating relative motion in a manner parallel to the sensing surface but not rotating around the center, which may significantly improve the ligand-analyte binding efficiency compared to the case without motion. The relative motion velocity in this motion mode does not change with the deviation of the center position of the sensor and sample solution, so that the consistency between different test results is better than that of the central rotational relative motion mode.

In an exemplary embodiment, a detection device is shown in FIG. 1. The detection device mainly includes a light source 10, a spectrometer 15, a directional coupler 11, and an optical fiber end facet sensor 14. The sensor is a sensor 14 having surface plasmon resonance (SPR) on the end facet of the optical fiber. The sensor 14 integrates a surface plasmon resonance (SPR) resonance cavity on the end facet of a 780nm-wavelength single-mode fiber. The resonant cavity includes two nanoslit arrays with different periods on a 55nm thickness golden film. The size of a central array is 11 µm× 11 µm, and the period of the nanoslit in the central array is 645 nm. The central array is surrounded by another array with a size of 100 µm × 100 µm, and a period of the nanoslit is 315 nm. The width of the nanoslit is 50nm, and the depth of the nanoslit penetrates the golden film. The sensing mode of this embodiment is shown in FIG. 1. The broad-spectrum light emitted by light source 10 of a super-luminescent diode enters the single-mode fiber (SMF) 12. The directional coupler 11 guides the broad-spectrum light to the surface plasmon resonance (SPR) sensor 14 on the end facet of the optical fiber. The optical fiber having a surface plasmon resonance (SPR) sensor 14 on the end facet is vertically inserted into a sample solution 13, the analyte is dispersed or dissolved in the sample solution 13. The light reflected back to the optical fiber by the sensor is then guided to a spectrometer 15 through a directional coupler 11 for reflection spectrum measurement. Due to the surface plasmon resonance (SPR), the reflection spectrum will have a resonance valley at approximately 850nm wavelength, and the central wavelength of the resonance valley red-shifts as the number of molecules adsorbed on the surface of the golded film increases. By measuring the central wavelength of the resonance valley, the binding and dissociation of the analyte to the surface of the golded film (sensing surface) can be obtained.

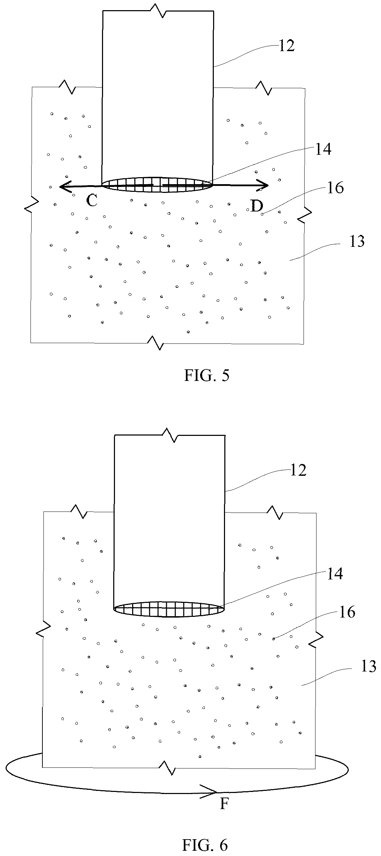

FIGs. 4-6 show the motion modes of the sensor and sample solution in the experiment. The sample solution is placed in a standard 96-well microtitre plate, and the sample solution in each well is 200µL. An optical fiber having a surface plasmon resonance (SPR) sensor on the end facet is vertically inserted into the wells and immersed into the sample solution, as shown in FIG. 1. The motion modes are selected from the following four:
1) as shown in FIG. 6, the sensor does not move, while the multi-well microtitre plate carries out a circular rotary oscillatory motion F, with a rotation diameter of about 1mm, a rotational frequency of about 1000rpm, and a total rotational speed of about 52mm/s;
2) as shown in FIG. 5, the multi-well microtitre plate does not move, while the optical fiber having the surface plasmon resonance (SPR) sensor on the end facet carries out horizontal reciprocating motions C and D, with a motion amplitude of 2mm, a motion frequency of 3Hz, and a total average motion velocity of 12mm/s;
3) as shown in FIG. 4, the multi-well microtitre plate does not move, while the optical fiber having the surface plasmon resonance (SPR) sensor on the end facet carries out perpendicular reciprocating motions A and B, with a motion amplitude of 2mm, a motion frequency of 3Hz, and a total average motion velocity of 12mm/s;
4) the multi-well microtitre plate and the optical fiber having the surface plasmon resonance (SPR) sensor on the end facet do not have relative motion.

In this embodiment, two label-free biomolecular interaction experiments are used as examples to compare the experimental effects of the above three different motion modes, including 1) PPB-SA molecular interaction experiment; 2) BPA-hIgG molecular interaction experiment. Details are as follows:

### 1) PPB-SA molecular interaction experiment:

The buffer used in this experiment is Hepes:
4-(2-hydroxyethyl)-1-piperazineethanesulfonic acid. The ligand on the sensing surface of the optical fiber end-facet SPR sensor is PPB molecule:
(poly-L-lysine)-(polyethylene glycol)-biotin. The sample solution (analyte solution) is SA molecule solution: streptavidin, the solvent is Hepes, and the concentration is 1 µg/mL.

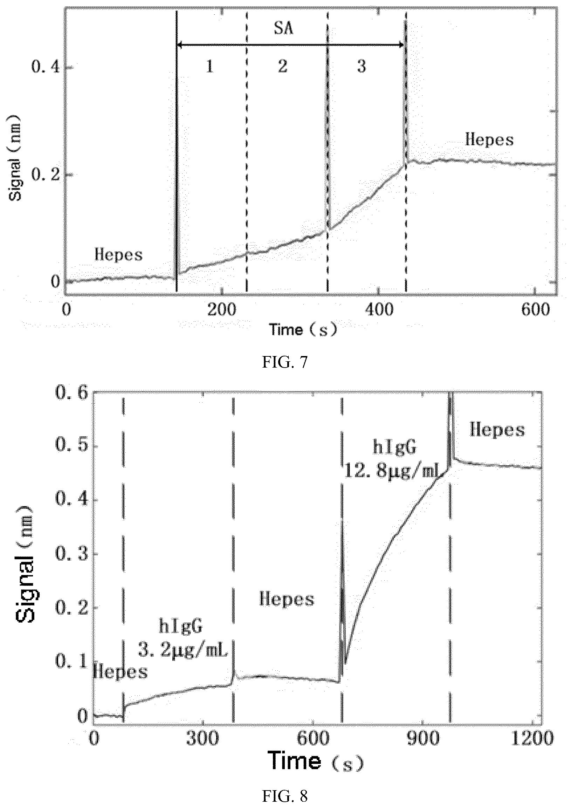

As shown in FIG. 7, the sensor is immersed into Hepes buffer, and neither the sensor nor the sample solution moves at this time. Then the sensor is immersed into the SA molecule solution to move in three motion modes in sequence, including: 1) the sensor does not move, and the multi-well microtitre plate carries out a circular rotary oscillatory motion F, with a rotation diameter of about 1mm, a rotational frequency of about 1000rpm, and a total rotational speed of about 52mm/s; 2) the multi-well microtitre plate does not move, and the optical fiber having the surface plasmon resonance (SPR) sensor on the end facet carries out horizontal reciprocating motions C and D, with a motion amplitude of 2mm, a motion frequency of 3Hz, and a total average motion velocity of 12mm/s; 3) the multi-well microtitre plate does not move, and the optical fiber having the surface plasmon resonance (SPR) sensor on the end facet carries out perpendicular reciprocating motions A and B, with a motion amplitude of 2mm, a motion frequency of 3Hz, and a total average motion velocity of 12mm/s. At last, the sensor is immersed into Hepes buffer, and neither the sensor nor the sample solution moves at this time. FIG. 7 shows the real-time process of the binding and dissociation of SA molecules to the sensor surface. The vertical axis is the variation amount of the central wavelength of the resonance valley in the SPR reflection spectrum, which has a linear relationship with the number of SA molecules binded to the sensing surface (the peak in FIG. 7 corresponds to a short time of the sensor from one well to another, and does not occur every time). As can be seen from FIG. 7, obviously, the perpendicular reciprocating motions of the optical fiber corresponds to a faster binding rate of SA molecules to the sensing surface. Although the speed of circular rotary oscillatory motion is much higher than the speed of horizontal reciprocating motion, the binding rate of SA molecules to the sensing surface has no significant difference.

### 2) BPA-hIgG molecular interaction experiment:

The buffer used in this experiment is Hepes:
4-(2-hydroxyethyl)-1-piperazineethanesulfonic acid. The ligand on the sensing surface of the optical fiber end-facet SPR sensor is BPA molecule: biotinylated protein A. The sample solution (analyte solution) is hIgG molecule solution: human immunoglobulin G, and the solvent is Hepes.

As shown in FIGs. 8-10, the sensor is immersed into Hepes buffer, hIgG solution with a concentration of 3.2 µg/mL, Hepes buffer, hIgG solution with a concentration of 12.8 µg/mL and Hepes buffer in sequence, and the whole measurement process maintains a same motion mode. FIGs. 8-10 respectively show measurement results under the following three motion modes: 1) the multi-well microtitre plate and the optical fiber having the surface plasmon resonance (SPR) sensor on the end facet do not move with respect to each other; 2) the sensor does not move, and the multi-well microtitre plate carries out a circular rotary oscillatory motion F, with a rotation diameter of about 1mm, a rotational frequency of about 1000rpm, and a total rotational speed of about 52mm/s; and 3) the multi-well microtitre plate does not move, and the optical fiber having the surface plasmon resonance (SPR) sensor on the end facet carries out perpendicular reciprocating motions A and B, with a motion amplitude of 2mm, a motion frequency of 3Hz, and a total average motion velocity of 12mm/s. As can be seen from FIGs. 8-10 that the binding rate of hIgG molecules to the sensing surface is greatly improved with the change of the motion mode from 1), 2) to 3). This improvement is even more significant in low-concentration hIgG solutions.

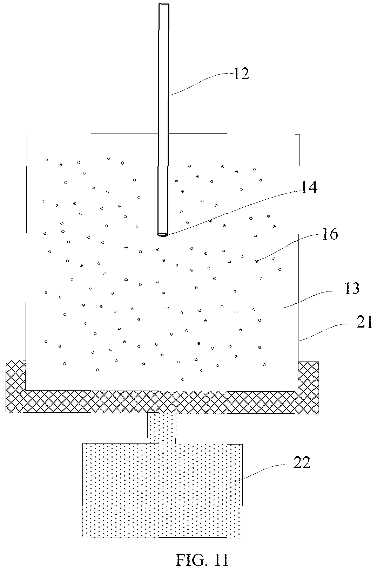

As shown in FIG. 11, this embodiment further provides a solid-phase surface and solution motion device, including: a container 21, the container 21 contains a solution 13, and a target object 16 is dissolved or dispersed into the solution 13; a solid-phase surface, the solid-phase surface includes a ligand in a biomolecular interaction and a sensor 14 for detecting the biomolecular interaction, the ligand is immobilized on a surface of the sensor 14, and the sensor 14 is inserted into the solution 13; and a drive motor 22, connected with the container 21, for implementing a reciprocating motion of the container 21. The reciprocating motion includes a motion direction perpendicular to the surface of the sensor 14, to improve at least one of the binding rate, dissociation rate, binding uniformity, binding directivity, and binding compactness of the target object 16 to the surface of the sensor 14.

In this embodiment, the reciprocating motion of the container 21 driven by the drive motor 22 further includes a motion direction parallel to the surface of the sensor 14. The drive motor 22 simultaneously drives the container 21 to move in a direction perpendicular to the surface of the sensor 14 and in a direction parallel to the surface of the sensor 14.

As an example, the drive motor includes a voice coil motor. The voice coil motor may generate regular motions through the interaction of magnetic poles in a magnetic field generated by a permanent magnetic field or an energized coil conductor, which can realize linear motion and a finite pendulum angle motion. Furthermore, compared with traditional flat-plate linear motors, the voice coil motor may provide better high-frequency response characteristics and is capable of making high-speed reciprocating motions.

As an example, the container includes a 96-well microtitre plate or a centrifuge tube.

As an example, the sensor includes one of a sensor on an optical fiber end facet and a sensor on an optical cable end facet. The end facet is approximately perpendicular to the optical wave transmission direction of the optical fiber or optical cable adjacent to the sensor. The sensor on an optical fiber end facet includes a sensor on the end facet of the optical fiber based on surface plasmon resonance, and the sensor on an optical cable end facet includes a sensor on the end facet of the optical cable based on biological membrane layer interference.

As described above, the solid-phase surface and solution motion mode and motion device of the present disclosure has the following beneficial effects:
1) The present disclosure provides a motion mode in which the relative motion of the sensing surface and the solution is perpendicular to the solid-phase surface. Compared with the traditional commonly used motion modes in which the relative motion is parallel to a sensing surface, the motion mode of the present disclosure can effectively improve the binding efficiency and dissociation efficiency of a ligand and an analyte at the same relative motion velocity of sensor and sample solution.
2) The perpendicular motion mode of the present disclosure will not cause significant unevenness in the flow rate of the sample solution, and may effectively improve the binding uniformity, binding directivity and binding compactness of the ligand to the analyte.
3) The present disclosure can effectively improve the detection sensitivity of biomolecular interactions, and therefore shows promises for a broad range of applications in biosensing detection.
4) When the perpendicular relative motion velocity of the sensor-sample solution is no less than 1/10 of the Brownian motion velocity of the analyte, such motion mode would significantly increase the probability of ligand-analyte collision. Therefore, for the measurement of the particle analyte with lower Brownian motion velocity and large mass, such as metallic particles, exosome particles, cells, quantum dots and medium particles, the present disclosure can remarkably increase the relative motion velocity of the analyte and the ligand.
5) The sensor-sample solution used in the present disclosure carries out a reciprocating relative motion in a manner parallel to the sensing surface but not rotating at the center, which may significantly improve the ligand-analyte binding efficiency compared to the case without motion. The relative motion velocity in this motion mode does not change with the deviation of the center position of the sensor and sample solution, so that the consistency between different test results is better than that of the central rotational relative motion mode.

Therefore, the present disclosure effectively overcomes various shortcomings in the traditional technology and has high industrial utilization value.

The above-described embodiments are merely illustrative of the principles of the disclosure and its effects, and are not intended to limit the disclosure. Modifications or variations of the above-described embodiments may be made by those skilled in the art without departing from the spirit and scope of the disclosure. Therefore, all equivalent modifications or changes made by those who have common knowledge in the art without departing from the spirit and technical concept disclosed by the present disclosure shall be still covered by the claims of the present disclosure.

## Claims

1. A solid-phase surface and solution motion mode, wherein during an interaction between the solid-phase surface and a target object, the target object is dissolved or dispersed into a solution, the solid-phase surface and the solution carry out a relative motion, and the relative motion includes a relative motion perpendicular to the solid-phase surface, to improve at least one of a binding rate, dissociation rate, binding uniformity, binding directivity, and binding compactness of the target object to the solid-phase surface.

2. The solid-phase surface and solution motion mode according to claim 1, wherein in binding and dissociating processes of the solid-phase surface and the target object, the relative motion between the solid-phase surface and the solution comprises a unidirectional forward motion perpendicular to the solid-phase surface, to improve at least one of binding rate, dissociation rate, binding uniformity, binding directivity, and binding compactness of the solid-phase surface to the target object.

3. The solid-phase surface and solution motion mode according to claim 1, wherein in binding and dissociating processes of the solid-phase surface and the target object, the relative motion of the solid-phase surface and the solution includes reciprocating forward motion and backward motion perpendicular to the solid-phase surface, to improve at least one of binding rate, dissociation rate, binding uniformity, binding directivity, and binding compactness of the target object and to solid-phase surface.

4. The solid-phase surface and solution motion mode according to claim 1, wherein the relative motion includes a combination of a relative motion perpendicular to the solid-phase surface and a relative motion parallel to the solid-phase surface.

5. The solid-phase surface and solution motion mode according to claim 4, wherein the relative motion perpendicular to the solid-phase surface and the relative motion parallel to the solid-phase surface are simultaneously performed.

6. The solid-phase surface and solution motion mode according to claim 4, wherein the relative motion parallel to the solid-phase surface includes one of a unidirectional parallel motion, a bidirectional reciprocating parallel motion, and a rotational parallel motion.

7. The solid-phase surface and solution motion mode according to claim 1, wherein the relative motion of the solid-phase surface and the solution includes one of only a motion of the solid-phase surface, only a motion of the solution, and simultaneous motions of the solid-phase surface and the solution.

8. The solid-phase surface and solution motion mode according to claim 1, wherein a velocity of a relative perpendicular motion of the solid-phase surface and the solution is no less than 1/10 of a Brownian motion velocity of the target object in the solution, to increase a collision probability of the solid-phase surface and the target object.

9. The solid-phase surface and solution motion mode according to claim 8, wherein the target object comprises a large-particle analyte, and the large-particle analyte is selected from a group consisting of a metallic particle, an exosome particle, a cell, a quantum dot, and a medium particle, or a combination thereof.

10. The solid-phase surface and solution motion mode according to claim 1, wherein the solid-phase surface comprises a ligand in a biomolecular interaction and a surface of a sensor for detecting the biomolecular interaction, the ligand is immobilized on the surface of the sensor, the target object includes an analyte in the biomolecular interaction, and the analyte is dissolved or dispersed into the solution; a binding and a dissociation of the target object to the solid-phase surface is a binding and a dissociation of the analyte to the ligand.

11. The solid-phase surface and solution motion mode according to claim 10, wherein the sensor includes one of a sensor on an optical fiber end facet and a sensor on an optical cable end facet, the end facet is approximately perpendicular to an optical wave transmission direction of an optical fiber or an optical cable adjacent to the sensor, the sensor on an optical fiber end facet includes a sensor on an end facet of an optical fiber based on surface plasmon resonance, and the sensor on an optical cable end facet includes a sensor on an end facet of an optical cable based on biological membrane layer interference.

12. A solid-phase surface and solution motion mode, wherein during an interaction between a solid-phase surface and a target object, the target object is dissolved or dispersed into a solution, the solid-phase surface and the solution carry out a relative motion, and the relative motion includes a non-central rotational reciprocating relative motion; a direction of the reciprocating relative motion is parallel to the solid-phase surface, to improve a binding rate and a dissociation rate of the target object to the solid-phase surface.

13. The solid-phase surface and solution motion mode according to claim 12, wherein the solid-phase surface comprises a ligand in a biomolecular interaction and a surface of a sensor for detecting the biomolecular interaction, the ligand is immobilized on the surface of the sensor, the target object includes an analyte in the biomolecular interaction, and the analyte is dissolved or dispersed into the solution; a binding and a dissociation of the target object to the solid-phase surface is a binding and a dissociation of the analyte to the ligand; the sensor includes a sensor on an optical fiber end facet or a sensor on an optical cable end facet, the end facet is approximately perpendicular to an optical wave transmission direction of an optical fiber or an optical cable adjacent to the sensor; the sensor on an optical fiber end facet includes a sensor on an end facet of an optical fiber based on surface plasmon resonance, and the sensor on an optical cable end facet includes a sensor on an end facet of an optical cable based on biological membrane layer interference.

14. A solid-phase surface and solution motion device, comprising:
a container, the container contains a solution, and a target object is dissolved or dispersed into the solution;
a solid-phase surface, the solid-phase surface includes a ligand in a biomolecular interaction and a sensor for detecting the biomolecular interaction, the ligand is immobilized on a surface of the sensor, and the sensor is inserted into the solution;
and a drive motor, connected with the container, for implementing a reciprocating motion of the container, the reciprocating motion includes a motion direction perpendicular to the surface of the sensor, to improve at least one of a binding rate, dissociation rate, binding uniformity, binding directivity, and binding compactness of the target object to the surface of the sensor.

15. The solid-phase surface and solution motion device according to claim 14, wherein the reciprocating motion of the container driven by the drive motor comprises a motion direction parallel to the surface of the sensor; the drive motor simultaneously drives the container to move in a direction perpendicular to the surface of the sensor and in a direction parallel to the surface of the sensor.

16. The solid-phase surface and solution motion device according to claim 14 or 15, wherein the drive motor comprises a voice coil motor.

17. The solid-phase surface and solution motion device according to claim 14, wherein the container comprises a 96-well microtitre plate or a centrifuge tube.

18. The solid-phase surface and solution motion device according to claim 14, wherein the sensor includes a sensor on an optical fiber end facet or a sensor on an optical cable end facet, the end facet is approximately perpendicular to an optical wave transmission direction of an optical fiber or an optical cable adjacent to the sensor, the sensor on an optical fiber end facet includes a sensor on an end facet of an optical fiber based on surface plasmon resonance, and the sensor on an optical cable end facet sensor includes a sensor on an end facet of an optical cable based on biological membrane layer interference.
